# FASCICULE DE BREVET EUROPEEN

(11) **EP 0 030 172 B1**
(45) Date de publication et mention de la délivrance du brevet: **09.02.1983**
(21) Numéro de dépôt: 80401540.2
(22) Date de dépôt: 30.10.1980
(51) Int. Cl.: A61K 39/00, A61K 35/18

(54) **Procédé de préparation d'antigène Rhésus, médicament et compositions pharmaceutiques contenant l'antigène et formes d'administration**
Verfahren zur Herstellung eines Rhesusantigens, Arzneimittel und pharmazeutische Zusammensetzungen, die das Antigen enthalten, und Verabreichungsformen
Process for preparing rhesus antigen, medicine and pharmaceutical compositions containing the antigen and administration forms

(30) Priorité: 31.10.1979 FR 7927080
(43) Date de publication de la demande: 10.06.1981
(73) Titulaire: OEUVRE REGIONALE DE TRANSFUSION SANGUINE DE TOULOUSE, 31052 Toulouse Cedex (FR)
(72) Inventeur: Bierme, Simone, F-31000 Toulouse (FR); Blanc, Madeleine, F-31000 Toulouse (FR); Abbal, Michel, F-31170 Tournefeuille (FR); Fournié, Alain, F-31300 Toulouse (FR)
(74) Mandataire: Hufénus, Christiane

## Description

L'invention a pour but la préparation, à partir de la membrane érythrocytaire, d'une substance antigénique Rh qui, administrée par voie orale à des femmes Rh-immunisées, diminue la réponse en anticorps de la classe IgG ou la remplace par une réponse locale en anticorps de la classe IgA, et en même temps produit une tolérance.

Il est bien connu que l'administration par voie orale d'un antigène à un animal (par exemple d'albumine humaine à un lapin) produit une forte réponse locale secrétoire en anticorps de la classe IgA, et en même temps une tolérance spécifique. L'expérience animale sur le système Rh-anti Rh est irréalisable puisqu'on ne retrouve l'anticorps en cause que dans l'espèce humaine. Mais d'autre part les travaux de De ASSIS chez l'homme sur l'extinction de la sensibilité tuberculinique par administration orale de l'antigène a montré la possibilité de supprimer une réaction immunitaire de façon spécifique. On a donc envisagé de stimuler, par administration orale d'une préparation d'un antigène de membrane Rh positif, les formations immunitaires intestinales dont la production pouvait interférer avec celle préexistante du système immunitaire parentéral et on a, dans un premier temps, contrôlé l'inocuité de cette préparation chez une volontaire Rh négatif immunisée.

L'invention a donc pour objet un procédé de préparation d'un produit antigénique à partir de globules rouges du groupe 0 Rh +, non utilisable en thérapeutique transfusionelle. Elle a également pour objet, à titre de médicament, le produit ainsi préparé.

Le produit selon l'invention est utile pour le traitement par voie orale, en cours de grossesse, des femmes Rh-immunisées. L'expérimentation clinique du produit a montré que celui-ci est capable de modifier la réponse de la mère vis-à-vis des antigènes Rh; il enraye la maladie en diminuant la production d'anticorps capables de traverser le placenta.

Six femmes portant des enfants Rh positif ont été traitées journellement, à partir de la 16e semaine de gestation, par administration orale du produit, associé éventuellement à de la prométhazine à hautes doses pour éviter l'hydrops foetalis et diminuer l'anémie foetale. Les femmes traitées avaient toutes des antécédents obstétricaux graves par immunisation Rh (mort in utero avec ou sans transfusions intra-utérines). Après le traitement par le produit selon l'invention, elles ont toutes donné naissance à des enfants vivants. Pendant tout le traitement et à des intervalles de deux semaines, on déterminait le titre en anticorps anti-Rh et les classes et sous-classes de ces anticorps. Les résultats étaient les suivants:
Le titre total d'anticorps n'augmentait pas pendant toute la gestation. Avant et pendant le premier trimestre, le sérum des femmes traitées contenait des anti-Rh de la sous-classe IgG, seulement. Ultérieurement, le titre en anticorps de cette sous-classe se stabilisait ou diminuait et une seconde sous-classe (IgG₃) apparaissait toujours à des titres faibles, et très variables. Chez trois des femmes auxquelles on avait administré des doses de produit 10 fois supérieures, de nouvelles classes d'anti-Rh (IgM et IgA) apparaissaient et, dans l'un des cas, les anticorps IgM persistaient associés à un faible taux d'IgG, pendant une année après la délivrance. Dans tous les cas, les bébés naissaient après environ 35 semaines de gestation dans de bonnes conditions et furent tous traités par exsanguino-transufsion post-natale.

Le procédé de préparation du produit selon l'invention consiste en une lyse hypotonique de globules rouges du groupe 0 Rh + dans un tampon phospho-calcique. Il est caractérisé par la succession des étapes suivantes:
- le culot de globules rouges, débarrassé du plasma et des globules blancs par centrifugation, est lavé avec une solution contenant un tampon P₀₄--, 1 M, pH 7,4 et du glucose;
- après élimination de la solution de lavage, les globules rouges sont remis en suspension dans une solution de CaC1₂, 0,025 M dans l'eau dé-ionisée. Après 1 heure environ, on obtient un précipité de stroma phospho-calcique dont la formation est accélérée et le rendement accru par addition de tampon phosphaté; on sépare le précipité par centrifugation;
- le stroma phospho-calcique est lavé avec la solution de lavage précédente, diluée avec de l'eau dé-ionisée, l'opération de lavage étant répétée plusieurs fois, jusqu'à obtenir une solution blanchâtre que l'on lyophilise.

La solution de lavage est constituée, en parties égales, par une solution tampon PO₄--, 1M, pH 7,4, et une solution à 5% de glucose. Le tampon est lui-même préparé à partir d'une solution de K₂HP0₄, 1 M (800 ml) et d'une solution de NaH₂P0₄, 1M (150 ml) pour obtenir un pH de 7,4. Ce tampon est préparé en eau distillée et ne contient ni EDTA, ni CINa.

La solution de CaC1₂ 0,025 M est préparée à partir de 50 ml de solution de CaC1₂, 1M, auxquels on ajoute deux litres d'eau dé-ionisée (c'est-à-dire d'eau distillée passée sur colonne de résine échangeur d'ions, d façon à être complètement dé-ionisée). L'eau distillée ne convient pas pour cette préparation.

L'exemple ci-après illustre de façon détaillée le procédé de préparation selon l'invention:

### Exemple

a) On centrifuge un culot de globules rouges non utilisables en thérapeutique transfusionelle à 1800 tr/min pendant 20 min pour éliminer le plasma et les globules bancs restants.

On prépare une solution de lavage avec des quantités égales de tampon phosphate 1M, pH 7,4 et d'une solution de glucose à 5%.

On place 200 ml de globules rouges dans une bouteille à sang de 500 ml et on les met en suspension dans une quantité suffisante de solution de lavage préparée comme ci-dessus, pour remplir la bouteille, et on centrifuge pendant 20 min à 1800 tr/min. Les globules blancs et la solution de lavage restants sont éliminés par aspiration. On effectue cette opération de lavage deux fois.

b) On prépare une solution de CaCl₂, 0,025 M à partir de 50 ml d'une solution de CaC1₂ 1 M dans 2 litres d'eau dé-ionisée. On ajoute cette solution de CaCl₂ aux globules rouges, de façon à obtenir un volume de 400 ml dans laquelle les globules rouges sont remis en suspension. On répartit cette solution entre quatre bouteilles de 500 ml (100 ml dans chaque bouteille). On remplit complètement ces bouteilles avec la solution de CaC1₂ 0,025 M (les bouteilles de sang de 500 ml contiennent environ 600 ml lorsqu'elles sont remplies complètement). Pour accélérer la précipitation, on ajoute quelques gouttes de la solution de lavage dans chaque flacon. Au bout de 45 min à 1 heure, le stroma phospho-calcique est complètement précipité. Si la précipitation semble cependant incomplète, les contenus de chaque bouteille sont répartis entre deux bouteilles et de nouveau dilués avec la solution de chlorure de CaC1₂ 0,02 M, de façon à remplir complètement la bouteille. Après précipitation, les diverses préparations sont centrifugées pendant 2 min à 2000 tr/min.

c) Après élimination du surnageant on mélange le précipité avec 10 ml de solution de lavage diluée par moitié avec de l'eau dé-ionisée, puis 150 ml d'eau dé-ionisée. On agite énergiquement les bouteilles pour remettre le précipité en suspension. Quand tout le stroma est détaché du fond de la bouteille, celle-ci est remplie jusqu'au repère 500 ml avec de l'eau dé-ionisée. On centrifuge la bouteille à 10°C pendant 1 à 2 min à 2000 tr/min. On élimine le surnageant et on répète ce lavage en agitant chaque fois le précipité énergiquement avec 10 ml de solution de lavage diluée et 150 ml d'eau dé-ionisée. Il faut environ 10 lavages pour obtenir une suspension blanchâtre. On lyophilise alors cette suspension blanchâtre.

L'antigène préparé selon le procédé de l'invention est présenté sous toute forme convenant à l'administration par voie orale De préférence, il est présenté sous forme de gélules ou de comprimés résistant au suc gastrique. L'enrobage gastro-résistant est constitué, de préférence, de trois couches d'une composition contenant par exemple de l'acéto-phtalate de cellulose (10 g) dissous dans le mélange acétone (100 ml) et chloroforme (23 ml).

Mais on peut utiliser toute autre préparation gallénique gastro-résistante compatible avec le produit.

Les gélules peuvent contenir de 100 à 500 mg de produit lyophilisé, et la dose à administrer est d'environ 2 g de lyophilisat par 24 heures répartis par exemple en 5 prises (absorbées à 6 heures, 10 heures, 14 heures, 18 heures et 22 heures). Le traitement débute à 16 semaines d'aménorrhée et se termine à l'accouchement, il a donc une durée moyenne de 18 semaines.

Les doses peuvent être augmentées jusqu'à 12 capsules de 400 mg par jour, et même jusqu'à 20 capsules par jour si les sous-classes IgG augmentent.

On peut associer le traitement avec le produit selon l'invention avec un traitement à la prométhazine: au bout de 16 semaines de grossesse on peut donner par exemple 6 comprimés de 25 mg de prométhazine (1 aux mêmes heures que le produit selon l'invention et 2 comprimés à 22 heures). Dans les formes graves, on peut passer directement à 11 comprimés par jour. A 26 semaines de gestation et jusqu'au terme, on donne de 16 à 20 comprimés de prométhazine par jour, aux mêmes heures que le produit selon l'invention (répartis de la façon suivante: 3, 3, 3, 3, 4 ou 4, 4, 4,4,4).

## Revendications

1. Procédé de préparation d'antigène Rhésus destiné à l'administration par voie orale, à partir de globules rouges du groupe 0 Rh positif, non utilisables en thérapeutique transfusionnelle, caractérisé par les étapes suivantes:
a) les globules rouges, séparés du plasma et les globules blancs, sont lavés avec une solution contenant un tampon phosphate, pH 7,4 et du glucose;
b) après élimination de la solution de lavage, les globules rouges sont remis en suspension dans une solution de CaC1₂, 0,025 M, dans l'eau dé-ionisée; après 1 heure environ, on obtient, par addition d'un peu de tampon de lavage selon a) un précipité de stroma phospho-calcique que l'on sépare par centrifugation;
c) le stroma phospho-calcique est lavé avec la solution de lavage selon a) diluée avec de l'eau dé-ionisée, et le lavage est répété plusieurs fois jusqu'à obtenir une solution blanchâtre que l'on lyophilise.

2. Procédé selon la revendication 1, caractérisé en ce que la solution de lavage est constituée, en parties égales, par une solution tampon PO₄--, 1 M, pH 7,4 et une solution à 5% de glucose.

3. Procédé selon l'une des revendications 1 et 2, caractérisé en ce que le tampon PO₄-- est préparé à partir d'une solution de phosphate dipotassique 1 M, et de phosphate monosodique 1 M, en eau distillée.

4. Procédé selon l'une des revendications 1 à 3, caractérisé en ce que la solution de CaC1₂, 0,025 M est préparée avec de l'eau dé-ionisée.

5. A titre médicament, pour utilisation dans le traitement des femmes Rh-immunisées et la prévention de l'immunisation Rh chez les femmes Rh-négatif, le produit préparé selon l'une quelconque des revendications 1 à 4.

6. Compositions pharmaceutiques, caractérisées en ce qu'elles comprennent le médicament selon la revendication 5, avec un excipient convenant à l'administration par voie orale.

7. Formes d'administration des compositions selon la revendication 6, telles que gélules, comprimés, caractérisées en ce qu'elles présentent un enrobage résistant au suc gastrique.

8. Formes selon la revendication 7, caractérisées en ce que l'enrobage gastro-résistant est constitué par de l'acéto-phtalate de cellulose dans un mélange d'acétone et de chloroforme.

## Patentansprüche

1. Verfahren zur Herstellung eines Rhesusantigens, bestimmt für die orale Verabreichung, ausgehend von roten Blutkörperchen der Gruppe 0 Rh positiv, die nicht bei der Blutübertragungstherapie verwendbar sind, gekennzeichnet durch folgende Stufen:
a) Waschen der roten Blutkörperchen, die aus Blutplasma und weißen Blutkörperchen abgeschieden wurden, mit einer Lösung, die einen Phosphatpuffer mit einem pH-Wert von 7,4 und Glucose enthält;
b) Suspendieren der roten Blutkörperchen nach Abtrennung der Waschlösung in einer 0,025-molaren CaCl₂-Lösung in deionisiertem Wasser; Zugabe nach ungefähr 1 Stunde einer geringen Menge an Waschpuffer gemäß a) unter Erhalt eines Niederschlags von Phospho-Calcium-Stroma, das man durch Zentrifugieren trennt;
c) Waschen des Phospho-Calcium-Stromas mit einer Waschlösung gemäß a), die mit deionisiertem Wasser verdünnt war, und mehrfach wiederholtes Waschen, bis man eine weißliche Lösung erhält, die man gefriertrocknet.

2. Verfahren nach Anspruch 1, dadurch gekennzeichnet, daß sich die eingesetzte Waschlösung aus gleichen Anteilen einer 1-molaren Phosphationenpufferlösung mit einem pH-Wert von 7,4 sowie einer 5%igen Glucoselösung zusammensetzt.

3. Verfahren nach Anspruch 1 oder 2, dadurch gekennzeichnet, daß man den Phosphationenpuffer aus einer 1-molaren Dikaliumphosphatlösung und aus einer 1-molaren Mononatriumphosphatlösung in destilliertem Wasser herstellt.

4. Verfahren nach einem der Ansprüche 1 bis 3, dadurch gekennzeichnet, daß man die 0,025-molare CaCl_{z}-Lösung mit deionisiertem Wasser herstellt.

5. Arzneimittel zur Anwendung bei der Behandlung von Rhesusfaktor-immunisierten Frauen und zur Vorbeugung der Rhesusfaktor-Immunität bei Frauen mit negativem Rhesusfaktor, wobei das Arzneimittel nach einem der Ansprüche 1 bis 4 hergestellt wurde.

6. Pharmazeutische Zusammensetzungen, dadurch gekennzeichnet, daß sie das Arzneimittel nach Anspruch 5 mit einer üblichen Trägersubstanz zur oralen Verabreichung enthalten.

7. Verabreichungsformen der Zusammensetzungen nach Anspruch 6, z. B. solche wie Gelkapseln oder Tabletten, dadurch gekennzeichnet, daß sie eine magensaftresistente Umhüllung aufweisen.

8. Verabreichungsformen nach Anspruch 7, dadurch gekennzeichnet, daß die magensaftresistente Umhüllung aus Cellulose-Acetophthalat in einer Mischung aus Aceton und Chloroform zusammengesetzt ist.

## Claims

1. A process for the preparation of Rhesus antigen for oral administration, from red corpuscles of the 0 Rh positive group not usable in transfusion therapeutics, characterized by the following steps:
a) the red corpuscles separated from plasma and the white corpuscles are washed with a solution containing a phosphate buffer, pH 7.4 and glucose;
b) after removal from the washing solution, the red corpuscles are suspended again in a solution of CaC1₂, 0.025 M, in de-ionized water; after about 1 hour, a phospho-calcium stroma precipitate is obtained which is separated by centrifugation;
c) the phospho-calcium stroma is washed with the washing solution according to a) diluted with de-ionized water and washing is repeated several times until a whitish solution is obtained which is lyophilized.

2. A process according to claim 1, characterized in that the washing solution consists in equal parts of a P0₄-- buffer solution, 1M, pH 7.4 and a 5% solution of glucose.

3. A process according to claims 1 and 2, characterized in that the P0₄- - buffer solution is prepared from a solution of mono acid potassium phosphate 1 M and diacid sodium phosphate 1 M in distilled water.

4. A process according to any one of claims 1 to 3, characterized in that the solution of CaC1₂, 0.025 M is prepared with de-ionized water.

5. The product prepared according to any one of claims 1 to 4 as a medicine for use in the treatment of Rh-immunized women and in the prevention of immunization in Rh-negative women.

6. Pharmaceutical compositions characterized in that they contain the medicine according to claim 5 and an excipient suitable for oral administration.

7. Compositions according to claim 6 prepared in the form of capsules, tablets, characterized in that they present a coating resistant to the gastric juice.

8. Compositions according to claim 7, characterized in that the coating resistant to gastric juice consists of cellulose aceto-phthalate in a mixture of acetone and chloroform.
